# EUROPEAN PATENT SPECIFICATION

(11) **EP 4 618 960 B1**
(45) Date of publication and mention of the grant of the patent: **09.12.2026**
(21) Application number: 23913277.2
(22) Date of filing: 26.12.2023
(51) Int. Cl.: C07D 413/02, A61K 31/12, A61K 31/515, A61P 35/00, C07D 239/66

(54) **SPECIFIC ARYLIDENE BARBITURATE DERIVATIVE MOLECULES FOR USE AS DRUG CANDIDATES IN CANCER TREATMENT**
SPEZIFISCHE ARYLIDENBARBITURATDERIVATMOLEKÜLE ZUR VERWENDUNG ALS WIRKSTOFFKANDIDATEN IN DER KREBSBEHANDLUNG
MOLÉCULES DE DÉRIVÉ D'ARYLIDÈNE BARBITURIQUE SPÉCIFIQUES DESTINÉES À ÊTRE UTILISÉES EN TANT QUE CANDIDATS-MÉDICAMENTS DANS LE TRAITEMENT DU CANCER

(30) Priority: 27.12.2022 TR 2022020743
(43) Date of publication of application: 24.09.2025
(73) Proprietor: Istanbul Medipol Universitesi, 34810 Beykoz/Istanbul (TR); Zonguldak Bulent Ecevit Universitesi, 67100 Incivez/Zonguldak (TR)
(72) Inventor: GERCEK, Zuhal, 67100 Incivez/Zonguldak (TR); YASIN, Betul, 34815 Istanbul (TR); GUZEL, Mustafa, 34815 Istanbul (TR)
(74) Representative: Yalçiner Patent and Consulting Ltd.
(86) International application number: PCT/TR2023/051693
(87) International publication number: WO 2024/144680

(56) References cited:
- WO-A1-2018/167187
- WO-A1-2019/043706
- RAJYALAKSHMI GUDIPATI; RAMA NARSIMHA REDDY ANREDDY; SARANGAPANI MANDA;: "Synthesis, characterization and anticancer activity of certain 3-{4-(5-mercapto-1,3,4-oxadiazole-2-yl)phenylimino}indolin-2-one derivatives", SAUDI PHARMACEUTICAL JOURNAL, ELSEVIER, AMSTERDAM, NL, vol. 19, no. 3, 29 January 2011 (2011-01-29), AMSTERDAM, NL , pages 153 - 158, XP028224162, ISSN: 1319-0164, DOI: 10.1016/j.jsps.2011.03.002
- LIAO YI-JEN, HSU SHIH-MING, CHIEN CHIA-YING, WANG YUAN-HSI, HSU MING-HUA, SUK FAT-MOON: "Treatment with a New Barbituric Acid Derivative Exerts Antiproliferative and Antimigratory Effects against Sorafenib Resistance in Hepatocellular Carcinoma", MOLECULES, SPRINGER VERLAG, BERLIN, DE, vol. 25, no. 12, 20 June 2020 (2020-06-20), DE , pages 2856, XP093195452, ISSN: 1433-1373, DOI: 10.3390/molecules25122856

## Description

### Technical Field

The present invention relates to specific Arylidene Barbiturate analogs that are effective on cancer and/or the mechanisms that cause this disease, to synthesis methods of said analogs/derivatives and to their use in the treatment of various diseases, especially cancer and cancer-causing diseases.

### State of the Art

Cancer is a multifactorial disease caused by uncontrolled proliferation, growth and spread of abnormal cells. Cancer follows cardiovascular diseases among the diseases that cause the highest number of deaths in the world. Neoplastic cells are first reduced by surgery or radiation therapy, or their progression is slowed down in treatment. These are followed by chemotherapy or immunotherapy. Cancer chemotherapy aims to prevent the growth and development of the tumor with drugs, as well as to provide cytotoxic effects by eliminating its re-development. There are studies in the literature showing that tyrosine kinase inhibitors (TKI) used for this purpose have an important place in cancer treatment by prolonging the life span of patients.

Most TKIs have undesirable properties such as variable bioavailability, poor water solubility, drug interaction, membrane transport and tissue permeability due to poor or irregular absorption. Therefore, it is necessary to develop new drug molecules with lower toxicity and higher oral bioavailability.

PCT application WO 2018/167187 discloses 5-carboxamide-2-thiobarbituric acids useful in the treatment of cancer.

### Brief Description of the Invention

In the invention, novel 1,3-bis(5-mercapto-1,3,4-oxadiazol-2-yl)-2-thioxo-dihydropyrimidie-4,6-(1H,5H)-dione derivatives were synthesized and their anti-cancer activities were investigated in vitro.

The anti-cancer activity of the synthesized novel molecules was determined by cell culture studies, and more deaths were detected in cancer cells than in normal cells. These results indicate that said drug candidate molecules may be less toxic in normal cells.

Molecules determined by anti-cancer activity in vitro activities, which may be new tyrosine kinase inhibitor candidates with the mentioned properties, were synthesized in this study.

### Definitions of Figures Describing the Invention

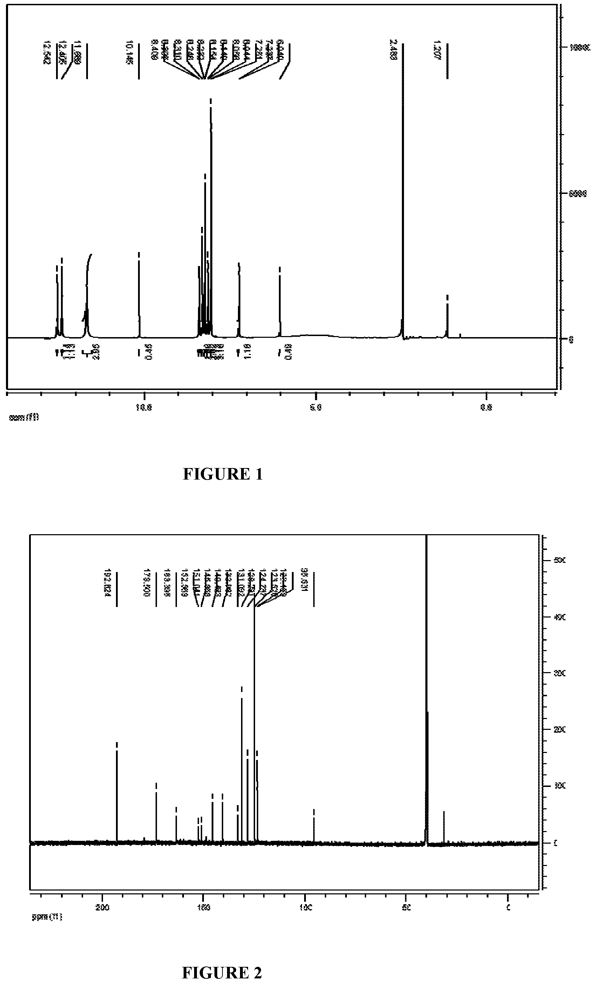

**Figure 1****:** ¹H NMR spectrum of 5-(4-nitrobenzylidene)-2-thioxo-dihydropyrimidine-4,6-(1H,5H)-dione.
**Figure 2****:** ¹³C NMR spectrum of 5-(4-nitrobenzylidene)-2-thioxo-dihydropyrimidine-4,6-(1H,5H)-dione.

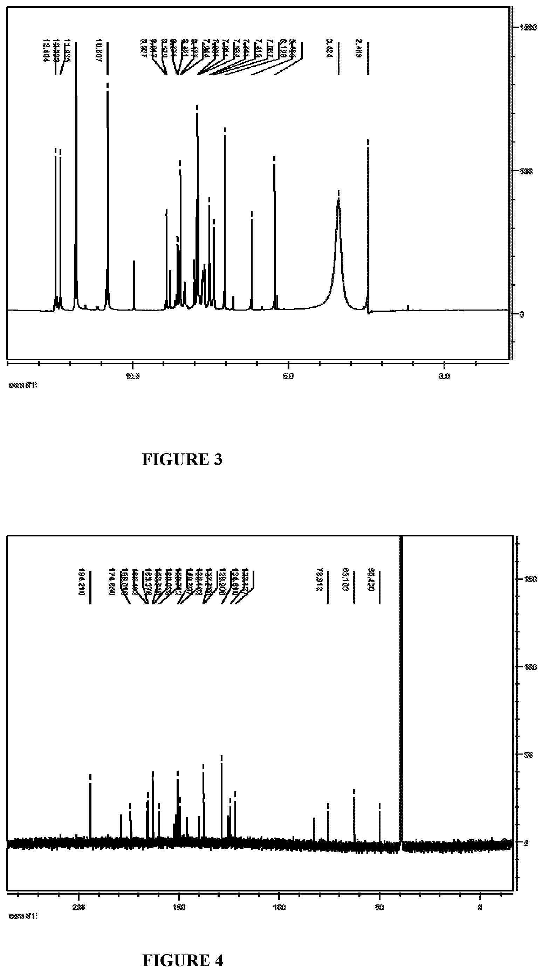

**Figure 3****:** ¹H NMR (DMSO-d6, 600 MHz) spectrum of Formula I.
**Figure 4****:** ¹³C NMR (DMSO-d6, 600 MHz) spectrum of Formula I.

### Detailed Description of the Invention

The present invention relates to Formula I, Formula II, Formula III and Formula IV compounds, the structural formulas of which are shown below.

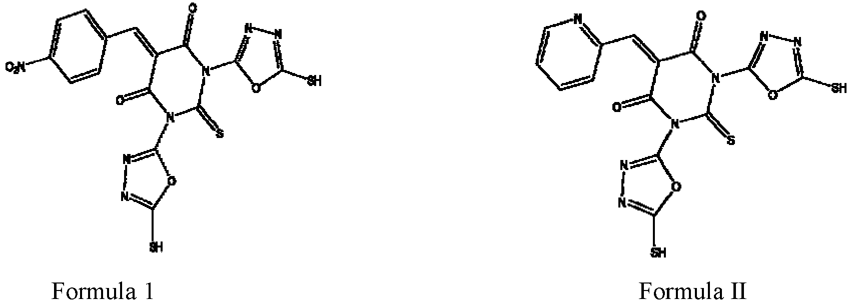

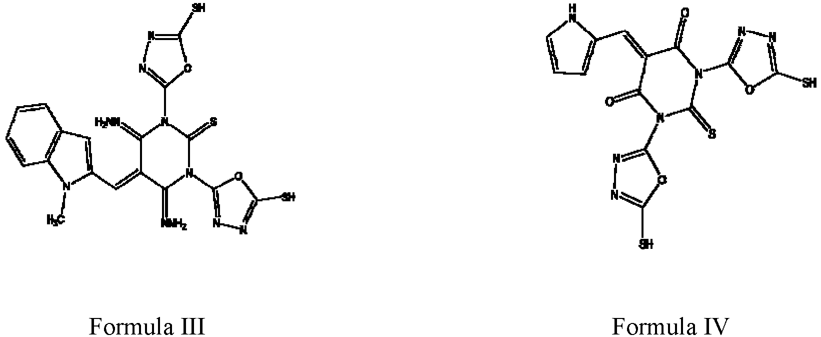

The molecules whose specific chemical structures are given above are given to explain the invention and the scope of the invention is not limited to these molecules. The invention also includes salts, hydrates, solvents, polymorphs, optical isomers, geometric isomers, enantiomers, diastereomers and mixtures thereof of these compounds.

Arylidene barbiturate derivatives were synthesized as a result of the reaction of different aldehydes with thiobarbituric acid in ethanol in the first step in the synthesis of the target compounds. The synthesis of 4-Nitro benzaldehyde derivative is shown as step I in the following.

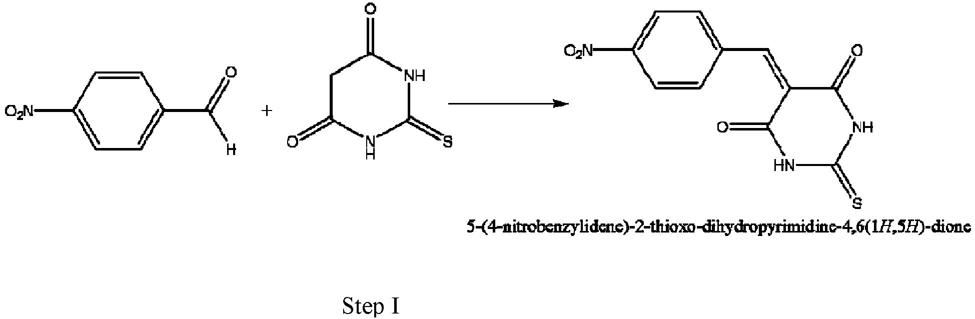

Solid potassium carbonate (2.5 eq) and ethylbromoacetate (2eq) are added to the arylidene barbiturate (1eq) mixed in N, N-dimethylformamide (20 ml) in the second step. The mixture is stirred at room temperature for 2-4 hours. It is then poured into 100 ml of water. The resulting solids 5-(4-nitrobenzylidene)-2-thioxo-N, N-acetate-pyrimidine-4,6-(1H,5H)-dione **(1.a)** are filtered.

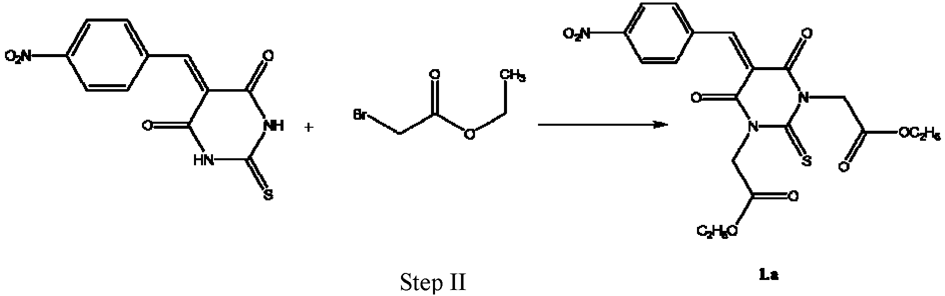

The product obtained in step II, **1.a,** is taken into methanol without further purification. 2 eq. hydrazine hydrate is added dropwise, and the mixture is stirred at room temperature for 3 hours. Then, water is added to it and the resulting precipitate **1.b** is filtered.

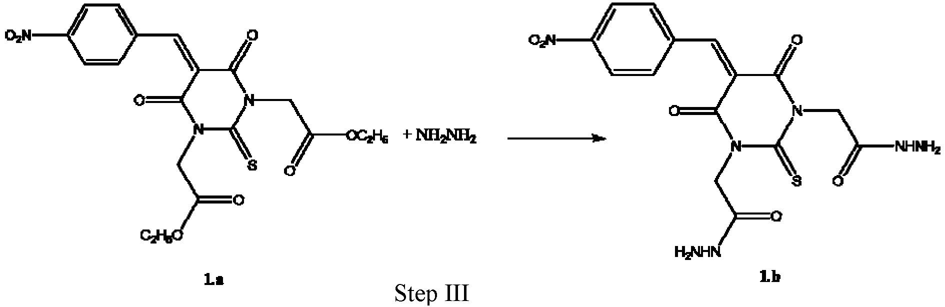

A mixture of 10 mmol potassium hydroxide, 10 mmol compound **1.b** and 15 mmol carbon disulfide in 50 mL of ethanol was heated at 80°C for 8 hours in step IV. After the reaction was completed, the solvent was evaporated in vacuum, and the reaction mixture was dissolved in ice water and acidified with dilute hydrochloric acid. The precipitate was filtered, water was passed, dried and recrystallized from ethanol to obtain **Formula I.** Structure ¹H NMR was verified with ¹³C NMR (84% efficiency)

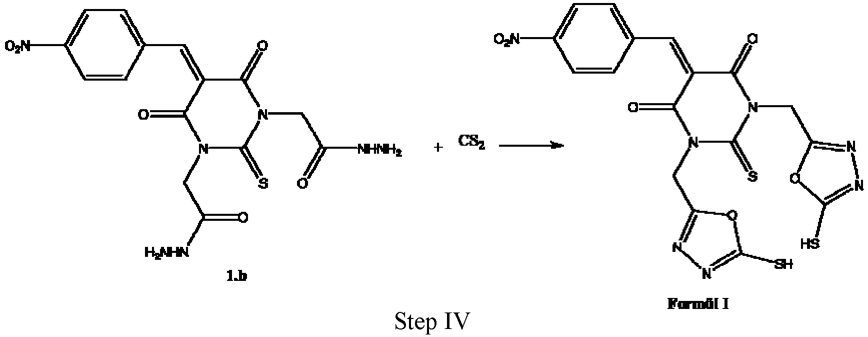

The above general synthesis steps are used for all proposed molecules. Diagram 1 shows the synthesis flow chart of compounds with Formula I, Formula II, and Formula III.

### Diagram 1:

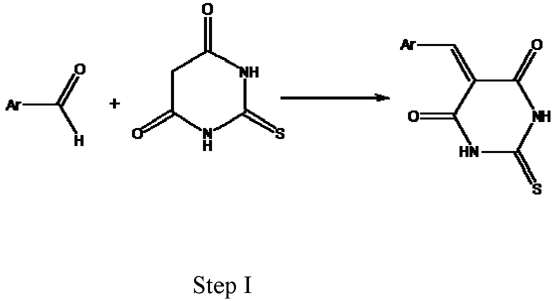

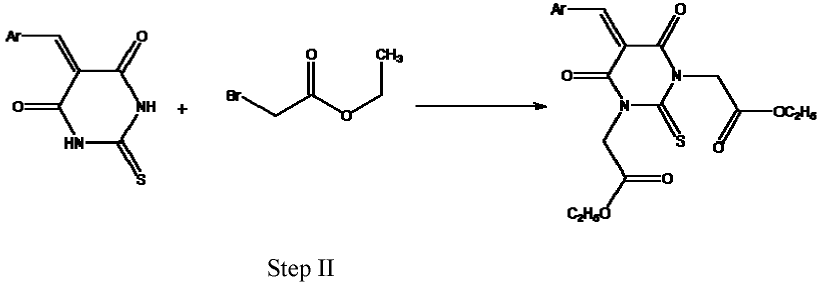

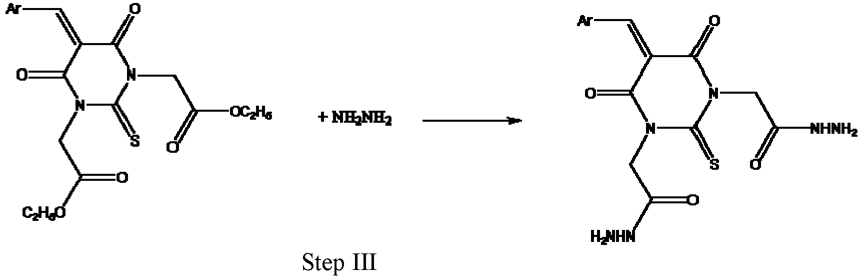

Ar.

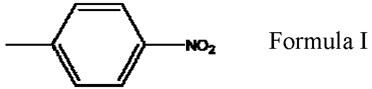

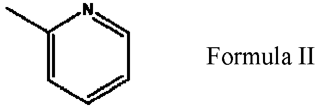

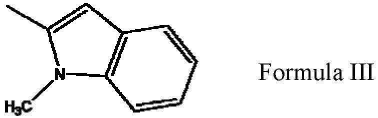

5-((1H-pyrrol-2-yl)methylene)-2-thioxo-dihydropyrimidine-4,6-(1H,5H)-dione **491** was synthesized as a result of the reaction of pyrolaldehyde with thiobarbituric acid in ethanol in the first step of the synthesis of the ZA-494 compound with Formula IV.

Solid potassium carbonate (2.5 eq) and ethylbromoacetate (2eq) are added to **491** (1eq) mixed in N, N-dimethylformamide (20 ml) in the second step. The mixture is stirred at room temperature for 2-4 hours. It is then poured into 100 ml of water. The resulting solids (5-(1H-pyrrol-2-yl)methylene)-2-thioxo-N, N-acetate-pyrimidine-4,6-(1H,5H)-dione **(492)** are filtered.

The product obtained in step 3, **492,** is taken into methanol without further purification. 2 eq. hydrazine hydrate is added dropwise, and the mixture is stirred at room temperature for 3 hours. Then, water is added to it and the resulting precipitate **493** is filtered.

A mixture of 10 mmol potassium hydroxide, 10 mmol compound **493** and 15 mmol carbon disulfide in 50 mL of ethanol was heated at 80°C for 8 hours in step 4. After the reaction was completed, the solvent was evaporated in vacuum, and the reaction mixture was dissolved in ice water and acidified with dilute hydrochloric acid. The precipitate was filtered, water was passed, dried and recrystallized from ethanol to obtain **494.** A schematic representation of the synthesis method of the ZA-494 compound is shown in Diagram 2.

### Diagram 2:

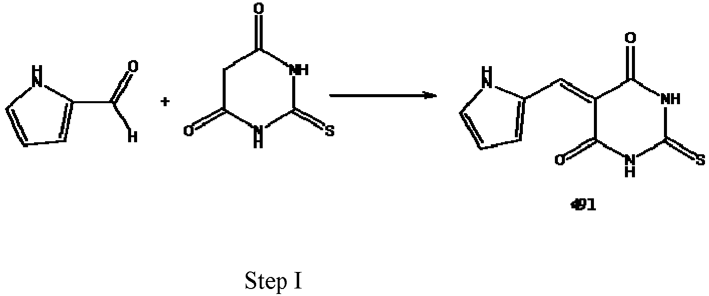

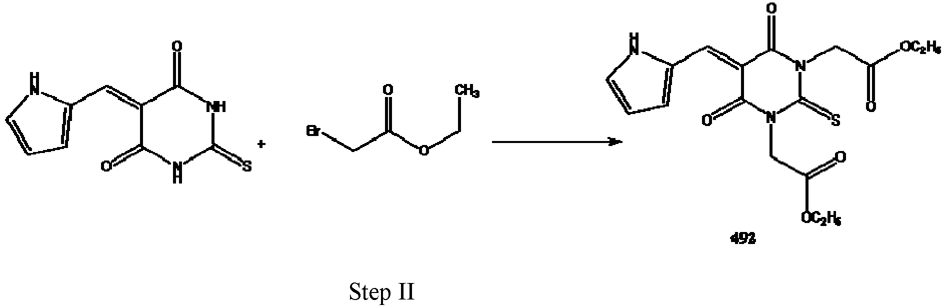

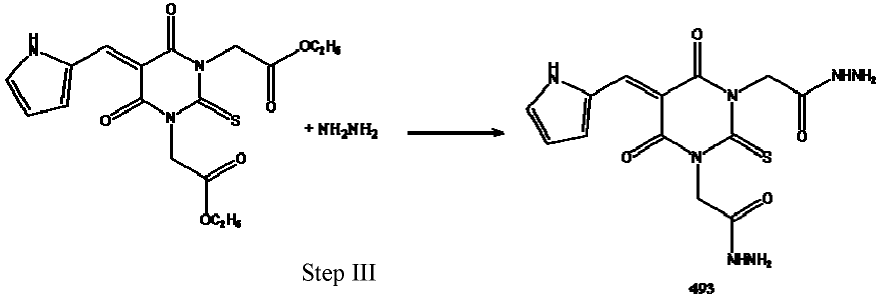

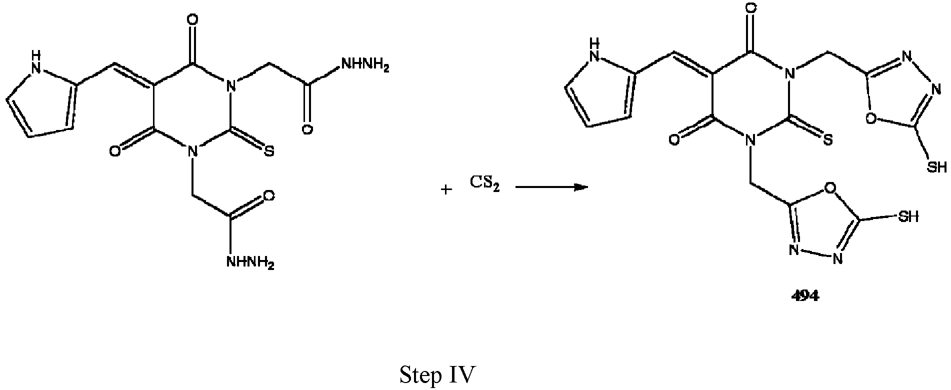

In-vitro anti-cancer biological activity of the molecules, various derivatives (ZA-494, ZA-514, ZA-544, ZA-554) of the molecules whose general structure are specified as Formula I, Formula II, Formula III, and Formula IV were tested in the breast cancer (MDA-MB-231) cell line and the Fibroblast (MRC-5) cell line, and the IC₅₀ values are given in Table 1 below. The maximal inhibitory concentration (IC₅₀) dose that kills half of the cells in the cancer cell line was found to be fairly low, and this value was found to be lower compared to normal cells (fibroblast) according to the results in the table. This indicates that there may be a candidate drug.

**Table 1**

| | | IC-50 (µM) IN MDA-MB 231 CELL LINE | IC-50 (µM) IN MRC-5 CELL LINE |
|---|---|---|---|
| 1. | Molecule (ZA-494) | 56.05 | 105.5 |
| 2. | Molecule (ZA-514) | 6.536 | 420 |
| 3. | Molecule (ZA-544) | 14.58 | 102.4 |
| 4. | Molecule (ZA-544) | 9.985 | 382.2 |

Activity studies of the molecules are ongoing. The results given are the first activity test results of some derivatives of the molecules whose general structure is given. The structures of these molecules are given in Table 2.

**Table 2**

| **Substance Code** | **Molecular Structure** |
|---|---|
| ZA-494 | 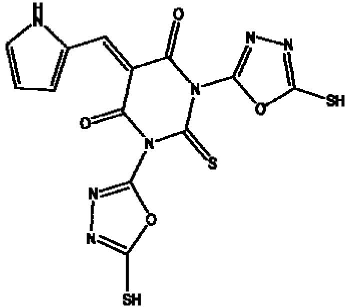 |
| ZA-514 (Formula I) | 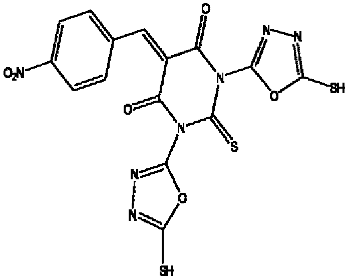 |
| ZA-544 (Formula II) | 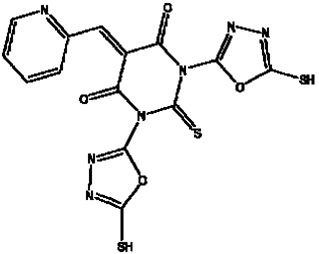 |
| ZA-554 (Formula III) | 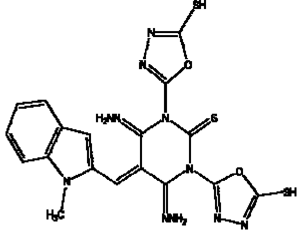 |

## Claims

1. A compound represented by Formula I, II, III or IV or pharmaceutically acceptable salt, solvent, hydrate, hydrate salt, polymorph, optical isomer, geometric isomer, enantiomer, diastereomer thereof.

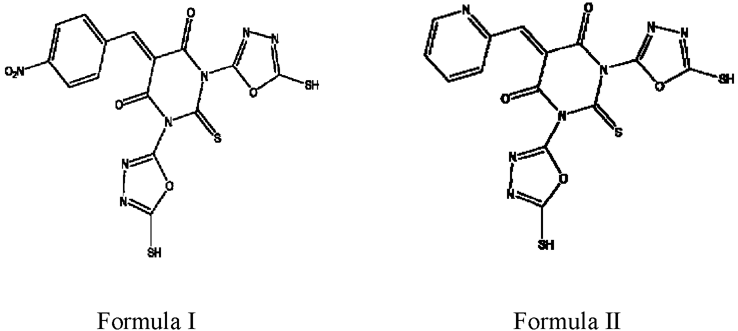

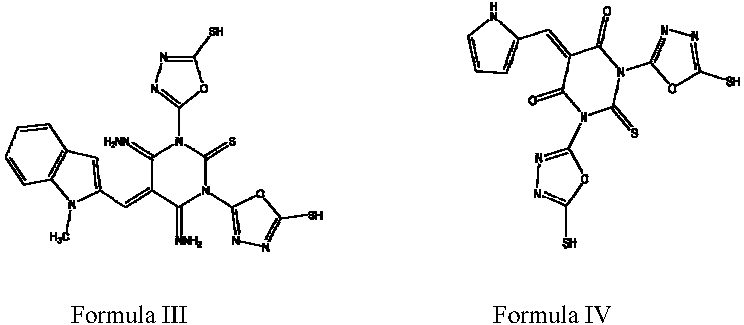

2. A compound or pharmaceutically acceptable salt, solvent, hydrate, hydrate salt, polymorph, optical isomer, geometric isomer, enantiomer, diastereomer thereof according to Claim 1 for use as a drug in the treatment of cancer disease.

3. A pharmaceutical composition, **characterized in that** it comprises the compound of according to Claim 1.

## Patentansprüche

1. Eine Verbindung, dargestellt durch die Formel I, II, III oder IV, oder ein pharmazeutisch verträgliches Salz, Lösungsmittel, Hydrat, Hydratsalz, Polymorph, optisches Isomer, geometrisches Isomer, Enantiomer, Diastereomer davon.

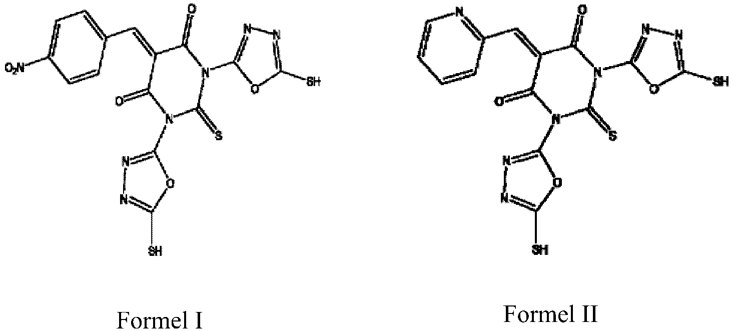

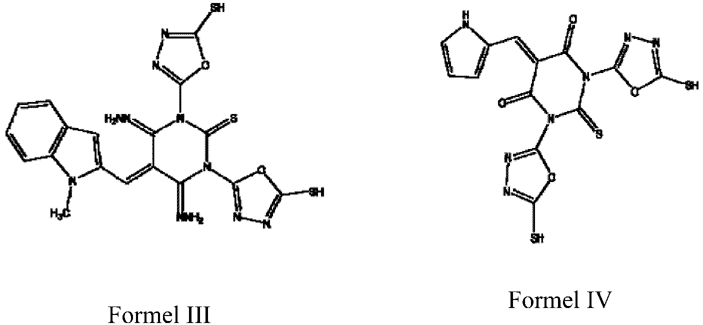

2. Eine Verbindung oder ein pharmazeutisch verträgliches Salz, Lösungsmittel, Hydrat, Hydratsalz, Polymorph, optisches Isomer, geometrisches Isomer, Enantiomer, Diastereomer davon nach Anspruch 1 zur Verwendung als Arzneimittel bei der Behandlung von Krebserkrankungen.

3. Eine pharmazeutische Zusammensetzung, **dadurch gekennzeichnet, dass** sie die Verbindung nach Anspruch 1 umfasst.

## Revendications

1. Composé représenté par les Formules I, II, III ou IV, ou sel pharmaceutiquement acceptable, solvant, hydrate, sel d'hydrate, polymorphe, isomère optique, isomère géométrique, énantiomère, diastéréoisomère de celui-ci.

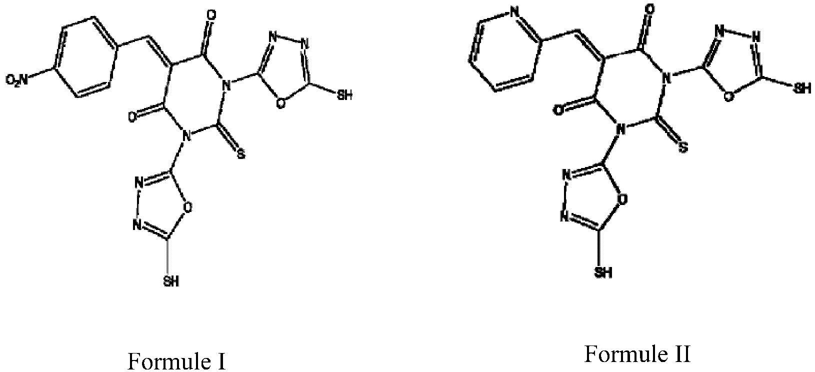

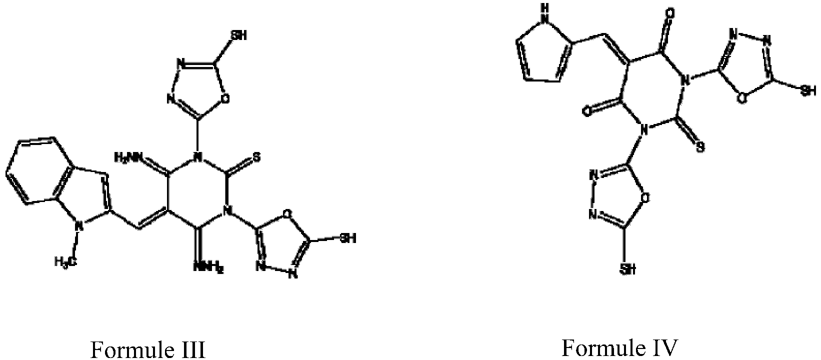

2. Composé ou sel pharmaceutiquement acceptable, solvant, hydrate, sel d'hydrate, polymorphe, isomère optique, isomère géométrique, énantiomère, diastéréoisomère de celui-ci selon la revendication 1 pour une utilisation en tant que médicament dans le traitement des maladies cancéreuses.

3. Composition pharmaceutique, **caractérisée en ce qu'**elle comprend le composé selon la revendication 1.
